# EUROPEAN PATENT APPLICATION

(11) **EP 3 470 000 A1**
(43) Date of publication of application: **17.04.2019**
(21) Application number: 18199049.0
(22) Date of filing: 08.10.2018
(51) Int. Cl.: A61B 17/70, A61B 17/88

(54) **APPARATUS FOR TREATING VERTEBRAL FRACTURES**

(30) Priority: 10.10.2017 US 201715728581
(71) Applicant: Globus Medical, Inc., Audubon, PA 19403 (US)
(72) Inventor: O'HALLORAN, Damien, CONSHOHOCKEN, PA 19428 (US); PAUL, David C., PHOENIXVILLE, PA 19460 (US); SUH, Sean, MORGANVILLE, NJ 07751 (US); CICCHINI, Stephen, NORTH WALES, PA 19454 (US); DORAN, Robert, EAGLEVILLE, PA 19403 (US)
(74) Representative: Morabito, Sara

(57) **Abstract**

Methods and apparatus for treating bones, including, in one or more embodiments, methods and apparatus for treatment of vertebral fractures that include an inflation device for cavity creation and an inflation and containment device for maintaining vertebral height and cement containment. Methods for treating a bone comprising: creating a cavity in the bone; inflating a containment jacket in the cavity; inflating a balloon within the containment jacket so that the balloon occupies a first portion of the containment jacket; introducing a first filler material into a second portion of the containment jacket, wherein the second portion of the containment jacket is not occupied by the balloon; removing the balloon from the containment jacket; and introducing a second filler material into the first portion of the containment jacket.

## Description

### CROSS REFERENCE TO RELATED APPLICATIONS

This application is a continuation-in-part application of U.S. Patent Application No. 15/355,100, filed on November 18, 2016 (published as U.S. Patent Publication No. 2017-0065309), which is a continuation of U.S. Patent Application Serial Number 14/254,614, filed on April 16, 2014 (now issued as U.S. Patent No. 9,526,550), which is a continuation of U.S. Patent Application Serial Number 12/632,325, filed on December 7, 2009 (now issued as U.S. Patent No. 8,734,458), all of which are incorporated by reference in their entirety herein.

### FIELD OF THE INVENTION

The present disclosure generally relates to treatment of bones. In particular, in one or more embodiments, the present disclosure relates to methods and apparatus for treatment of vertebral fractures that include an inflation device for cavity creation and an inflation and containment device for maintaining vertebral height and cement containment.

### BACKGROUND

Bones and bony structures are susceptible to a variety of weaknesses that can affect their ability to provide support and structure. Weaknesses in bony structures may have many causes, including degenerative diseases, tumors, fractures, and dislocations. By way of example, weaknesses in vertebrae from lead to compression fractures that involve the collapse of one or more vertebrae in the spine. These vertebral compression fractures may be caused by a number of conditions including osteoporosis, trauma, and tumors. Advances in medicine and engineering have provided doctors with a plurality of devices and techniques for alleviating or curing these weaknesses.

One technique for treating vertebral fractures is vertebroplasty. In vertebroplasty, a physician may use a needle to inject bone cement into a fractured vertebral body to stabilize the fracture. Kyphoplasty is another technique for treating vertebra fractures that involves insertion of a balloon into the fractured vertebra to create a bone cavity in the vertebra. The balloon may then be removed followed by injection of bone cement into the vertebral body to stabilize the fracture. Leakage of the bone cement in both vertebroplasty and kyphoplasty is a common problem that can lead to complications. Another problem associated with these techniques is the potential for inadequate height restoration to the fractured vertebral body.

Thus, there is a need for methods and apparatus that can provide stabilization to a fractured vertebra.

### SUMMARY

The present disclosure generally relates to treatment of bones. In particular, in one or more embodiments, the present disclosure relates to methods and apparatus for treatment of vertebral fractures that includes a device for cavity creation, an inflation device and a containment device for maintaining vertebral height and cement containment.

An embodiment of the present invention includes a method for treating a bone. The method may comprise creating a cavity in the bone. The method further may comprise placing a containment jacket in the cavity. The method further may comprise inflating a balloon within the containment jacket so that the balloon occupies a first portion of the containment jacket. The method further may comprise introducing a first filler material into a second portion of the containment jacket, wherein the second portion of the containment jacket is not occupied by the balloon. The method further may comprise removing the balloon from the containment jacket. The method further may comprise introducing a second filler material into the first portion of the containment jacket.

According to a further aspect of the invention it is provided an implantable device for repairing a vertebral body, said implantable device comprising: a balloon implant having a neck portion and configured to receive bone cement, wherein the balloon implant has a larger size than the cavity; a shaft attached to the neck portion at a distal end; a hub attached to shaft at a proximal end; and a filler needle disposed inside the shaft and configured to provide bone cement to the balloon implant.

In a version the shaft further comprises a pair of steel tubes and the neck portion is disposed between the steel tubes and wherein the balloon implant is released from the shaft by retracting one of the pair of steel tubes.

In another version the shaft is attached to the neck portion via threaded connection.

In a further version, the shaft is configured to rotate to release the balloon implant from the threaded connection.

The features and advantages of the present invention will be readily apparent to those skilled in the art. While numerous changes may be made by those skilled in the art, such changes are within the spirit of the invention.

### BRIEF DESCRIPTION OF THE DRAWINGS

FIG. 1 is a cross-sectional view of a vertebral body in accordance with one embodiment of the present technique.
FIG. 2 illustrates an inflation device inserted into a vertebral body in accordance with one embodiment of the present invention.
FIG. 3 illustrates employment of an inflation device to create a cavity in a vertebral body in accordance with one embodiment of the present invention.
FIG. 4 illustrates a cavity created in a vertebral body in accordance with one embodiment of the present invention.
FIG. 5 illustrates employment of an inflation and containment device in a vertebral body in accordance with one embodiment of the present invention.
FIG. 6 illustrates further employment of an inflation and containment device in a vertebral body in accordance with one embodiment of the present invention.
FIG. 7 illustrates a stabilized vertebral body in accordance with one embodiment of the present invention.
FIG. 8 illustrates a containment jacket in accordance with one embodiment of the present invention.
FIG. 9 is a close-up view of a containment jacket in accordance with one embodiment of the present invention.
FIGS. 10 and 11 illustrate an inflation and containment device in accordance with one embodiment of the present invention.
FIG. 12 illustrates employment of an inflation and containment device in accordance with one embodiment of the present invention.
FIG. 13 illustrates further employment of an inflation and containment device in accordance with one embodiment of the present invention.
FIG. 14 illustrates yet further employment of an inflation and containment device in accordance with one embodiment of the present invention.
FIGS. 15-21 illustrate yet another embodiment of a system and method for treating vertebral fractures.
FIGS. 22A-29 illustrate another embodiment of a system for treating vertebral fractures.

### DETAILED DESCRIPTION OF THE ILLUSTRATED EMBODIMENTS

The present disclosure generally relates to treatment of bones. In particular, in one or more embodiments, the present disclosure relates to methods and apparatus for treatment of vertebral fractures that include an inflation device for cavity creation and an inflation and containment device for maintaining vertebral height and cement containment.

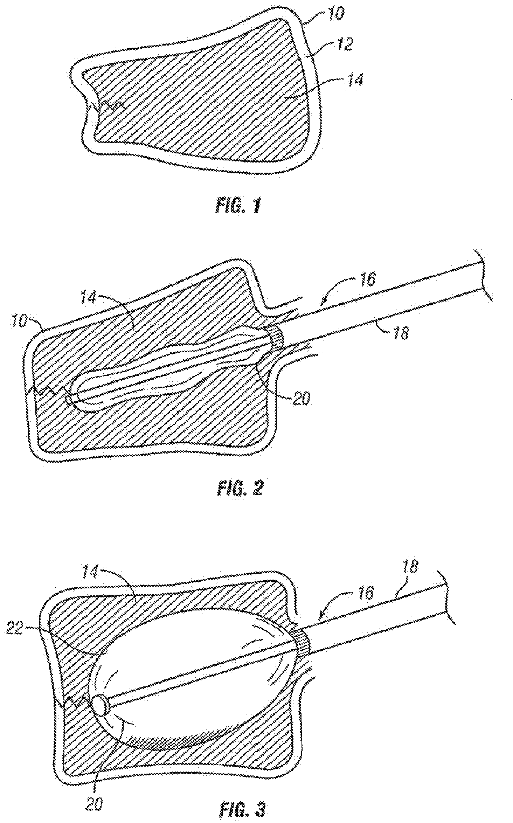

FIG. 1 illustrates a vertebral body 10 having a compression fracture therein with associated loss of height. As illustrated, the vertebral body 10 includes an exterior portion of cortical bone 12 and an interior portion of cancellous bone 14.

FIGS. 2-4 illustrate creation of a cavity in the vertebral body 10, in accordance with one embodiment of the present invention. As illustrated in FIG. 2, an inflation device 16 may be inserted into the cancellous bone 14 of the vertebral body 10. In the illustrated embodiment, the inflation device 16 includes a fill tube 18 and a first balloon 20 on the distal end of the fill tube 18. As illustrated, the first balloon 20 may be deflated when the inflation device 16 is inserted into the vertebral body 10. In certain embodiments, the inflation device 16 may be an inflatable bone tamp. It should be understood that the passageway into the vertebral body 10 for the inflation device 16 may be created using any of a variety of different suitable techniques. While not illustrated, a trocar, for example, may be used to place a cannula into the patient's body. A drill may then be inserted into the cannula, for example, and used to create a channel into the vertebral body 10 into which the inflation device 16 may be inserted. The inflation device 16 may then be inserted through the cannula into the vertebral body 10, for example.

FIG. 3 illustrates employment of the inflation device 16 to create a cavity 22 in the vertebral body 10, in accordance with one embodiment of the present invention. As illustrated, the first balloon 20 may inflate, for example, to compact the cancellous bone 14 in the interior portion of the vertebral body 10. In addition to creation of the cavity 22, the first balloon 20 may also, for example, force apart the compact bone 12, restoring height to the vertebral body 10. The inflation device 16 may then be removed from the vertebral body 10. While FIG. 3 illustrates the use of the first balloon 20 for creation of the cavity 22, those of ordinary skill in the art will appreciate that other suitable techniques may also be used for creation of the cavity 22. By way of example, an expandable jack or other suitable device may be used to create the cavity 22 in the vertebral body 10.

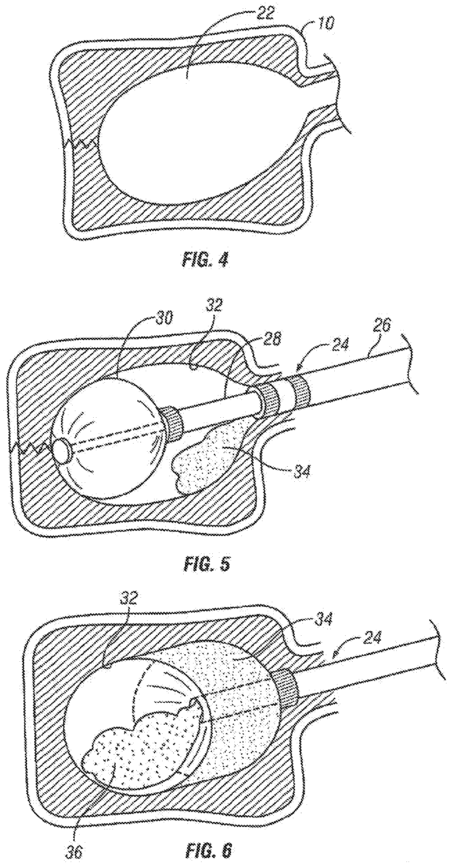

FIG. 4 illustrates the cavity 22 that has been created in the vertebral body 10 after removal of the inflation device 16, in accordance with embodiments of the present invention. While not illustrated, embodiments of the present invention further may include coating the wall of the cavity 22 with a bone growing agent, or a hemostatic sealing agent.

FIG. 5 illustrates employment of an inflation and containment device 24 in the vertebral body, in accordance with embodiments of the present invention. As illustrated, the inflation and containment device 24 may be inserted into the cavity 22 in the vertebral body 10. In certain embodiments, a second inflation device may be inserted into the vertebral body 10 through a cannula (not illustrated). In the illustrated embodiment, the inflation and containment device 24 includes an exterior tube 26, an interior fill tube 28, a second balloon 30, and a containment jacket 32. As will be discussed in more detail below, the second balloon 30 may be employed to maintain the height of the vertebral body 10 while the cavity 22 is partially filled with a first volume 34 of a filler material. The containment jacket 32 may be employed to contain a filler material (e.g., cement) introduced into the cavity 22 to prevent undesirable leakage. In this manner, problems associated with leakage of the filler material from the cavity 22 and loss of vertebral height may be reduced or possibly even avoided.

The second balloon 30 may be located on a distal end or proximal end of the interior fill tube 28, in accordance with embodiments of the present invention. In certain embodiments, the second balloon 30 and interior fill tube 28 may be an inflatable bone tamp. While not illustrated, the second balloon 30 is deflated when the inflation and containment device 24 is inserted into the vertebral body 10. After insertion into the cavity 22, the second balloon 30 may then be inflated. In general, inflation of the second balloon 30 should provide pressure on the walls of the cavity 22 to prevent (or reduce) loss of vertebral height. It may be desirable, in certain embodiments, for expansion of the second balloon 30 to further increase the height of the vertebral body 10. In certain embodiments, inflation of the second balloon 30 may restore some vertebral height lost after removal of the first balloon 20. As illustrated, the second balloon 30 generally may be enclosed within the containment jacket 32. The volume of the second balloon 30 generally should be smaller than the volume of the containment jacket 32, in accordance with embodiments of the present invention. Furthermore, when inflated, the second balloon 30 generally should not occupy the entire volume of the containment jacket 32. By way of example, the second balloon 30 may occupy from about 10% to about 90% by volume of the containment jacket 32.

The containment jacket 32 may be located on a distal end of the exterior tube 26, in accordance with embodiments of the present invention. As illustrated, the containment jacket 32 may be attached to the distal end of the exterior tube 26 such that the containment jacket 32 encloses the distal end of the exterior tube 26. While not illustrated, the containment jacket 32 may be deflated when the inflation and containment device 24 is inserted into the vertebral body 10. After insertion into the cavity 22, the containment jacket 32 may be inflated as the second balloon within the containment jacket is inflated. As illustrated, the containment jacket 32 may conform to the shape of the cavity 22. In certain embodiments, the volume of the containment jacket 32 may be larger than the volume of the cavity 22. It may be desirable, in certain embodiments, for the containment jacket 32 to be a compliant balloon (e.g., polyurethane) that can contain the filler material to prevent leakage. Accordingly, the containment jacket may permit interdigitation of the filler material with the cancellous bone, in accordance with embodiments of the present invention.

As illustrated by FIG. 5, employment of the inflation and containment device 24 includes inflation of the containment jacket 32 and inflation of the second balloon 30, in accordance with embodiments of the present technique. As further illustrated by FIG. 5, the cavity 22 may then be partially filled with a first cement volume 34. In the illustrated embodiment, the first cement volume 34 may be introduced into the containment jacket 32, for example, by way of the exterior tube 26. The first cement volume 34 generally may fill the portion of the containment jacket 32 that is not occupied by the inflated second balloon 30. By way of example, the first cement volume 34 may occupy from about 10% to about 90% by volume of the containment jacket 32 as inflated in the cavity 22. The first cement volume 34 may then be allowed to cure in the containment jacket 32. After the first cement volume 34 has substantially cured, the second balloon 30 may be removed from the cavity.

FIG. 6 illustrates further employment of the inflation and containment device 24 in accordance with embodiments of the present invention. As previously mentioned, the second balloon 30 may be removed from the cavity 22 after the first cement volume 34 has substantially cured. In the illustrated embodiment, the containment jacket 32 remains in the cavity 22. As illustrated, a second cement volume 36 may then be introduced into the containment jacket 32. The second cement volume 36 generally may occupy the unoccupied portion of the containment jacket 32, for example, the portion of the containment jacket 32 that is not occupied by the first volume 32 of cement. By way of example, the second cement volume 36 may occupy from about 10% to about 90% by volume of the containment jacket 32 as inflated in the cavity 22. The second cement volume 36 may then be allowed to cure in the containment jacket 32. The inflation and containment device 24 may then be removed from the vertebral body 10. In accordance with embodiments of the present invention, the containment jacket 32 may be detached from the device 24 and remain in the vertebral body 10.

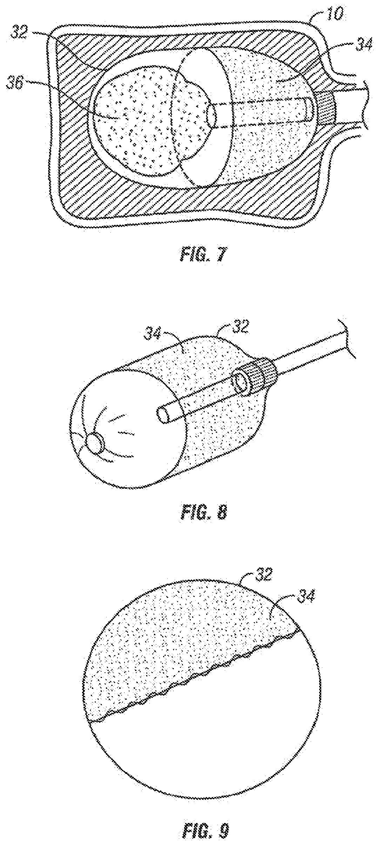

FIG. 7 illustrates the vertebral body 10 after stabilization of the compression fracture therein in accordance with embodiments of the present invention. As illustrated, the containment jacket 32 remains in the vertebral body 10 generally filling the cavity 22. The first cement volume 34 and the second cement volume 36 generally fill the containment jacket 32. As is readily apparent from a comparison of FIG. 7 and FIG. 1, height may be restored to the vertebral body 10 in accordance with embodiments of the present invention.

While the preceding description of FIGS. 5-7 describes the use of the inflation and containment device 24, it should be understood that the inflation and containment device 24 is an illustration of one device for maintaining vertebral height and cement containment in accordance with embodiments of the present technique. Other suitable devices for maintaining vertebral height and cement containment may also be used in present embodiments.

FIGS. 8-9 illustrate the containment jacket 32 in accordance with one embodiment of the present invention. FIG. 9 is a close-up view of the containment jacket 32 in accordance with one embodiment of the present invention. As illustrated, the outer surface of the containment jacket 32 may be coated with a bone growing agent. The bone growing agent may be any of a variety of different materials suitable for promoting growth of the cancellous bone 14 that is adjacent to the containment jacket 32 in the vertebral body 10.

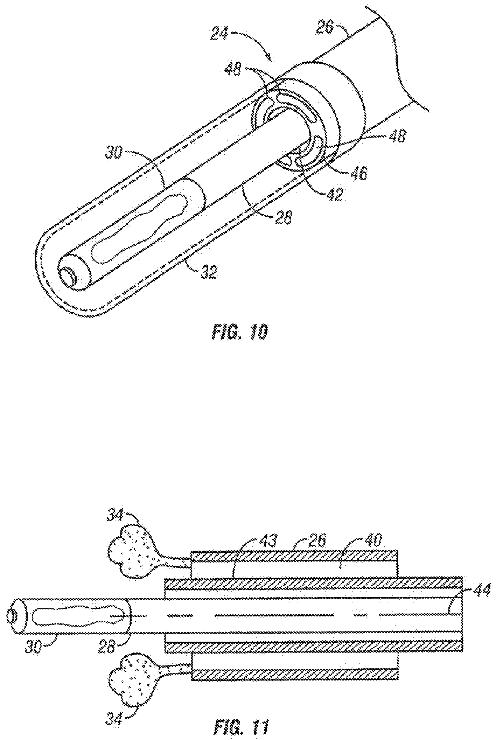

FIGS. 10-11 illustrate an inflation and containment device 24 that may be used in accordance with one embodiment of the present invention. As previously mentioned, the device 24 may be inserted into a vertebral body 10. As illustrated, the inflation and containment device 24 may comprise an exterior tube 26, an interior fill tube 28, a second balloon 30 in a deflated state, and a containment jacket 32. In the illustrated embodiment, the exterior tube 26 is a dual-duct tube that comprises an exterior passageway 40 an interior passageway 42. The exterior passageway may surround the interior passageway 42 with the passageways separated by an interior wall 43. Both the exterior passageway 40 and the interior passageway 42 may, for example, extend along the longitudinal axis 44 of the exterior tube. As illustrated, the distal end 46 of the exterior tube 26 may include one or more exit ports 48 for the exterior passageway 40. The exit ports 48 may be spaced around the interior passageway 42. The interior passageway 42 may be used to deliver, example, the first cement volume 34 into the containment jacket 32. The interior fill tube 28 may be disposed through the interior passageway 42 of the exterior tube 26.

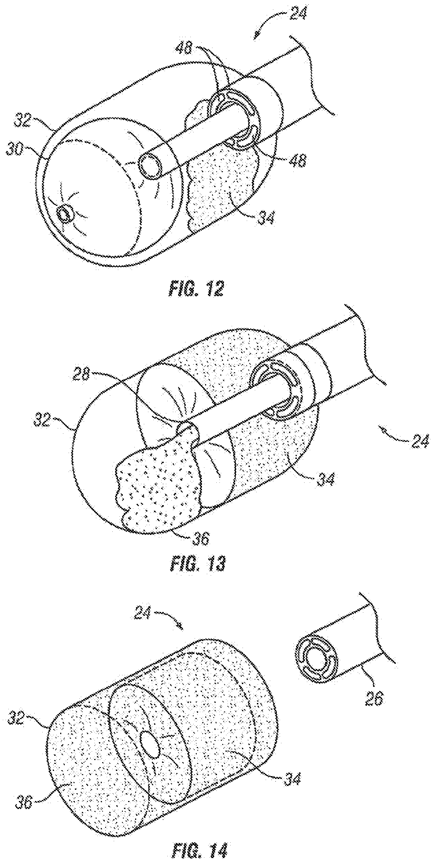

FIG. 12 illustrates employment of the inflation and containment device 24 in accordance with one embodiment of the present invention. As illustrated, employment of the device 24 may comprise inflating the second balloon 30 and the containment jacket 32. Embodiments of the present invention further may comprise introduction of a first cement volume 34 into the containment jacket 32. As illustrated, the first cement volume 34 may be introduced into the volume of the containment jacket 34 that is not occupied by the second balloon. The first cement volume 34 may be introduced through the exterior passageway 40 of the exterior tube 26, exiting into the containment jacket 34 from the one or more exit ports 48. A sufficient amount of the first cement volume 34, for example, may be introduced to generally fill the unoccupied portion of the containment jacket 34. The first cement volume 34 may then be allowed to cure in the containment jacket 34.

FIG. 13 illustrates further employment of the inflation and containment device 24 in accordance with one embodiment of the present invention. After the first cement volume 34 is substantially cured, for example, the second balloon 30 may be deflated and removed from the containment jacket 32. As illustrated, employment of the device 24 further may comprise introduction of the second cement volume 36 into the containment jacket 32. By way of example, the second cement volume 36 may be introduced into the space within the containment jacket 32 that was previously occupied by the second balloon 30 in an inflated state. A cement filling tube may be used to deliver the second cement volume 36 to the containment jacket 34. In certain embodiments, a sufficient amount of the second cement volume 36 may be introduced to substantially fill the remainder of the containment jacket 32. The second cement volume 36 may then be allowed to cure in the containment jacket 34.

FIG. 14 illustrates yet further employment of the inflation and containment device 24 in accordance with one embodiment of the present invention. As illustrated, the first cement volume 34 and the second cement volume 36 generally fill the containment jacket 32. As previously mentioned, the containment jacket 32 contains the cement volumes preventing undesirable leakage. The exterior tube 26 may then be detached from the containment jacket 32, leaving the containment jacket 32 in place. It should be noted that accessing the vertebral body may be achieved through the use of a cannula or through an open access method.

The preceding description describes the use of a first cement volume 34 and a second cement volume 36 in accordance with embodiments of the present invention. Those of ordinary skill in the art will appreciate that the first cement volume 34 and the second cement volume 36 may comprise any of a variety of bone cements suitable for use in orthopedic applications. An example of a suitable bone cement comprises polymethyl methacrylate (PMMA). In addition, while the preceding description describes the use of cement, embodiments of the present invention also may encompass a variety of different filler materials that may be utilized to, for example, fill and stabilize the cavity 22 in the vertebral body 10. Examples of suitable materials may include human bone graft and synthetic derived bone substitutes.

In the exemplary embodiment, a first balloon is used to create a cavity in the vertebral body. However, any device that can be used to create cavity may be applied. For example, mechanical devices such as stents, drills, and vacuums may be used to create a cavity in the vertebral body. After the cavity is created, a containment jacket and the second balloon can be introduced according to the present invention. It should also be noted that various different methods of deploying the second balloon within the containment jacket may be used. For instance, the second balloon may be positioned in any position to maximize the efficiency and ease for inserting the cement into a particular position in the vertebral cavity.

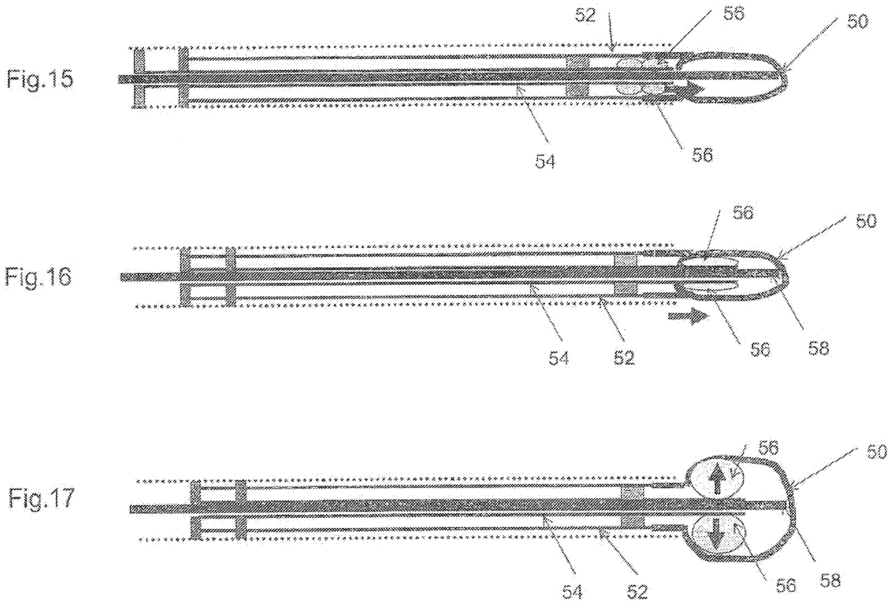

In another exemplary embodiment of the present invention, FIGS. 15-21 illustrate a containment jacket 50 attached to a distal end of an insertion device 52. The insertion device 52 is adapted to be coupled to the containment jacket 50. The insertion device 52 is also provided with a central port 54 that is used to insert medical devices in the cavity of the vertebral body and is contained within the containment jacket 50. For instance, the central port 54 can be used to insert a k-wire, endoscopic devices, bone cement and other medically compatible instrument to enhance the procedure of curing bone cement within the vertebral cavity. On opposing sides of the central port 54 and at the distal end of the central port 54, multiple balloons 56 may be attached. In different embodiments, the multiple balloons 56 may vary in size and shape. Also, on opposing sides of the central port 54 and between the insertion device walls 52, there is provided sealing elements 57. The sealing elements 57 are utilized to eliminate any risk of bone filler material or any other material being withdrawn through the insertion device 52.

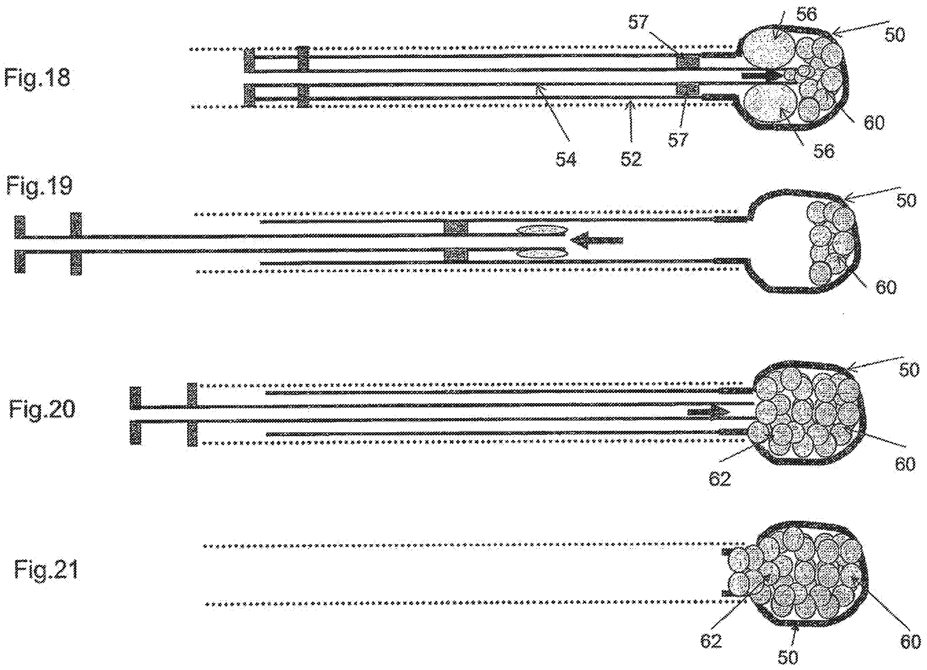

A central guide wire 58 is used to initially insert the containment jacket 50 within the vertebral body. Once the containment jacket 50 is positioned within the vertebral body as illustrated in FIG. 16, the multiple balloons 56 are inserted into the cavity and inflated as illustrated in FIGS. 16 and 17. As the balloons 56 are inflated, the pressure from the outer walls of the balloons 56 inflate the containment jacket 50. After the balloons 56 are inflated to a volume that is optimal, a first filler material 60 is then inserted through the central port to a first portion within the containment jacket 50. The balloons 56 are then deflated and removed from the containment jacket through the central port. Turning now to FIGS. 18 and 19, after removal of the balloons 56, the first filler material 60 is cured, thereby providing stability and restoring height to one portion of the of the containment jacket within the vertebral body. Once the first filler material 60 is cured, a second filler material 62 may be inserted into a second portion and allowed to cure within the containment jacket 50, as shown in FIG. 20. FIG. 21 illustrates the first and second filler material being contained within the containment jacket 50. Also, the containment jacket 50 conforms to the shape of the surrounding cancellous bone and interdigitates with the surrounding bone without any leakage of the first or second filler material.

In another embodiment of the present invention, a cavity creation balloon and a height restoration balloon may be configured within a containment jacket attached to a vertebral body insertion device. In one step of the present invention, containment jacket, the cavity creation balloon and the height restoration balloon are inserted into a fractured vertebral body. Next, the cavity creation balloon and the height restoration balloon are inflated simultaneous, thereby inflating the containment jacket within the vertebral body. Once the cavity is created and the height restoration balloon is inflated, the cavity creation balloon is deflated and removed. After removal of the cavity creation balloon, a bone filler material insertion device is inserted into the containment jacket and filled with a bone filler material in a first portion of the containment jacket. Once the bone filler material is cured, the height restoration balloon is removed and a second bone filler material is inserted into the second portion of the containment jacket. Next, the second bone filler material is cured and the insertion devices are removed, leaving the containment jacket containing the first and second bone filler material in the vertebral body.

In addition, the preceding description is directed, for example, to treatment of vertebral fractures that includes an inflation device for cavity creation and an inflation and containment device for maintaining vertebral height and cement containment. It should be understood that the present technique also may be used in other suitable bone treatments were maintenance of vertebral height and/or cement containment may be desired. By way of example, embodiments of the present invention may be used to treat tibia plateau fractures, distal radius fractures, and cancellous fractures.

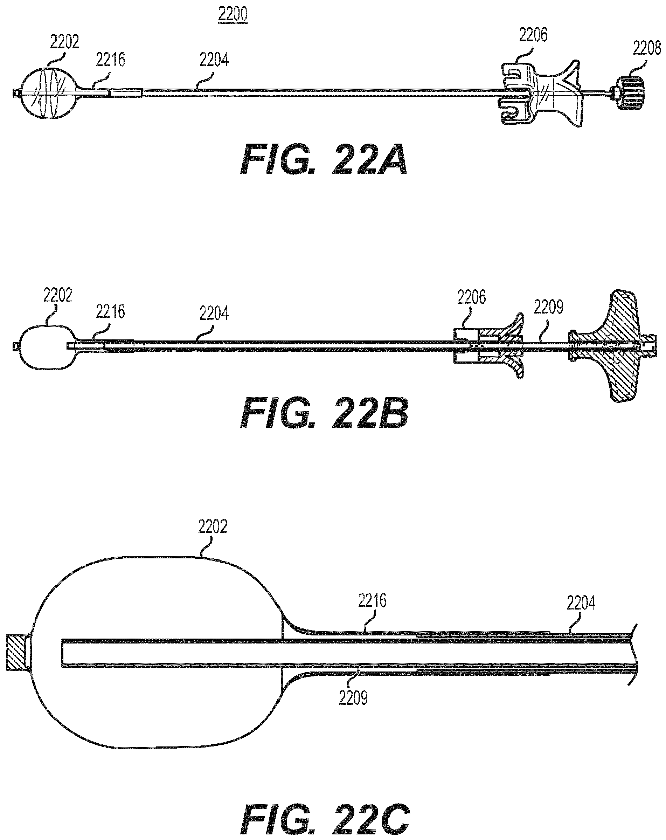

FIGS. 22A-29 illustrate an embodiment consistent with the principles of the present disclosure. Implantable device 2200, as described in further detail below, may comprise a biocompatible balloon implant which can be inserted into a cavity of a vertebral body through a cannulated access system. After insertion, the balloon may be filled with bone cement and conform to the boundaries of the cavity. Implantable device 2200 may lower the chance of cement extravasation by containing the bone cement within itself. The balloon implant, which may be attached to a shaft of implantable device 2200, can be detached after the cement cures, which leaves only the cement filled balloon in the vertebral body.

FIG. 22A illustrates an implantable device 2200 consistent with the principles of the present disclosure. Implantable device 2200 may include a balloon implant 2202, a shaft 2204, a hub 2206, and insertion mandrel 2208. Insertion mandrel 2208 may function to add rigidity to implantable device 2200 during insertion into the cavity. After insertion, balloon implant 2202 may be filled with bone cement.

As shown in FIG. 22B, implantable device 2200 is provided with a filler needle 2209 through which bone cement may be introduced into balloon implant 2202. FIG. 22C illustrates another view of filler needle 2209 being inserted into balloon implant 2202. Implantable balloon 2202 may be physically attached to shaft 2204 or implantable balloon 2202 and shaft 2204 may comprise a single unitary component. Bone cement may be provided through filler needle 2209 to fill balloon 2202. For example, filler needle 2209 may be placed through shaft 2204 to balloon implant 2202 allowing deposition of bone cement. Filler needle 2209 may have laser markings on the outside to aid in determining the correct depth for injecting bone cement. The diameter of filler needle 2209 may be determined such that the outside diameter of filler needle 2209 may have a clearance in relation to the inside diameter of shaft 2204 to allow for the venting of air, but not viscous liquids. This may aid in allowing for balloon implant 2202 to void entirely of air while being filled with bone cement.

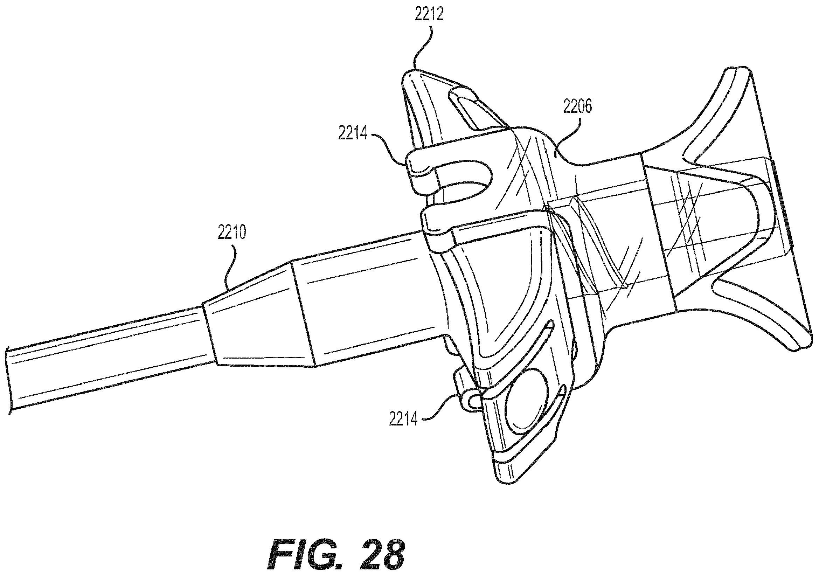

Hub 2206 is shown in greater detail in Fig. 28. Hub 2206 may lock onto an access cannula 2210, which may have a handle 2210. This configuration may prevent rotation or axial movement of the implantable device 2200. As shown in Fig. 28, wings 2214 of hub 2206 overlap handle 2210 and detents on hub 2206 may securely hold hub 2206 to handle 2210.

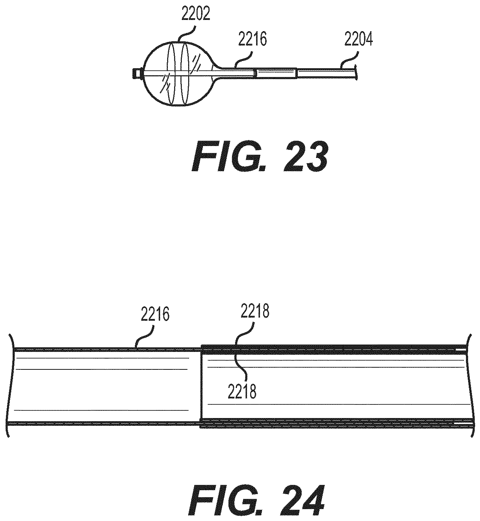

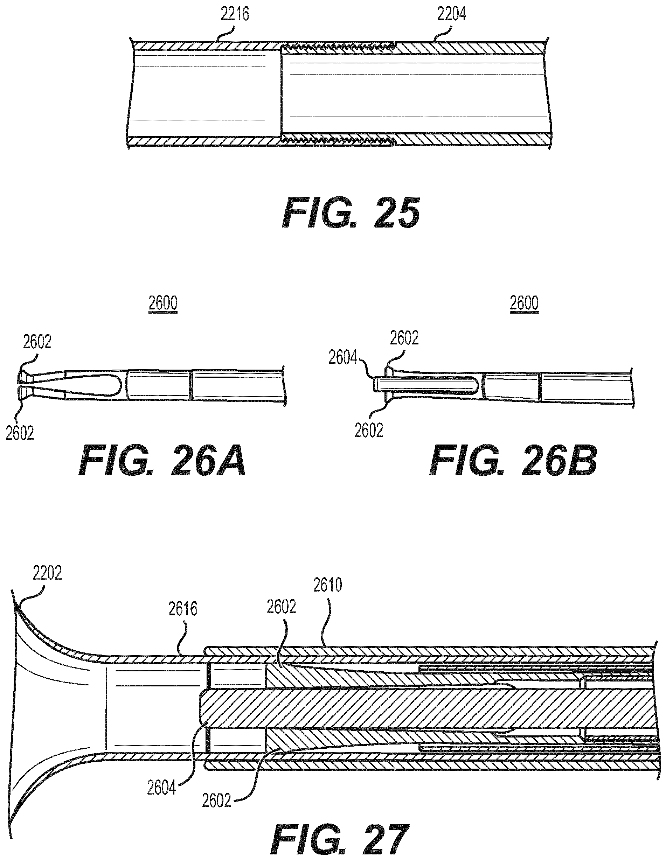

FIG. 23 illustrates balloon implant 2202 rigidly attached to shaft 2204. In this manner, a neck 2216 of balloon implant 2202 may be bonded to shaft 2204. Detachment of balloon implant 2202 from shaft 2204 is detailed with respect to FIGs. 26A-27.

FIGS. 26A and 26B illustrate a detachment device 2600, which may include jaws 2602. Detachment device 2600 may be a cannulated shaft, having multiple jaws 2602, that is inserted into shaft 2204 and extends to neck 2216. A mandrel may be inserted into the detachment device 2600 through its cannulation and force jaws 2602 to expand, as shown in FIG. 27.

FIG. 27 illustrates balloon implant 2202, neck 2216, cannula 2210, jaws 2602 of device 2600, and mandrel 2604. Upon insertion of mandrel 2604 through the cannulation of the detachment device 2600, jaws 2602 are forced to expand outwards towards the inner surface of cannula 2210, which interferes with the surface. Because of this, jaws 2602 cut through neck 2216 of balloon implant 2602 and detachment device 2600 is rotated to fully sever neck 2216 and allow it and balloon implant 2202 to remain in the vertebral body.

In another configuration, balloon implant 2202 may be releasably attached to shaft 2204. In FIG. 24, shaft 2204 may comprise two steel tubes 2218. Balloon implant 2202 may be disposed in a manner such that neck 2216 may be tightly sandwiched between steel tubes 2218. In order to release balloon implant 2202 from shaft 2204, one of steel tubes 2218 may be retracted thereby detaching balloon implant 2202 from shaft 2204.

FIG. 25 illustrates another manner in which balloon implant 2202 may be released from shaft 2204. Neck 2216 may be engaged with shaft 2204 through a threaded connection. In order to release balloon implant 2202 from shaft 2204, a user may rotate shaft 2204 until the threaded connection is undone thereby detaching balloon implant 2202 from shaft 2204.

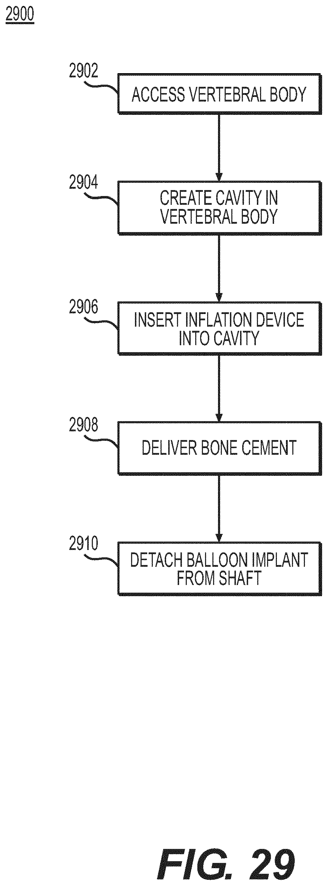

FIG. 29 illustrates a method 2900 for delivering bone cement to a vertebral body consistent with the present disclosure. At step 2902, using access instrumentation and fluoroscopic guidance, a working cannula and introducer is used to access the desired vertebral body. At step 2904, after accessing the vertebral body, a scraper, curette, bone tamp, or any combination of the aforementioned may be used with fluoroscopic guidance to create a cavity within the cancellous bone of the vertebral body. The cavity size may correspond to the size of the balloon implant being used. The implant size may be designed to be larger than the cavity created such that the implant balloon does not stretch during cement filling. At step 2906, after selecting an appropriately sized implant, the user may insert the inflation device through the cannula. At step 2908, bone cement is first prepared by mixing the liquid and powder components. The cement may be injected though a filler needle. The filler delivery needle may be inserted through the shaft of the inflation device, into the balloon implant under fluoroscopic guidance. At step 2910, after the cement has cured within the balloon implant, the balloon implant may be separated through detachment, unthreading, or separation and left to remain within the vertebral body.

While it is apparent that the invention disclosed herein is well calculated to fulfill the objects stated above, it will be appreciated that numerous modifications and embodiments may be devised by those skilled in the art.

The invention could be inter alia defined by the following examples:
1. A method for repairing a vertebral body, said method comprising: accessing the vertebral body via a cannula; creating a cavity within cancellous bone of the vertebral body; inserting an implantable device into the cavity via the cannula, wherein the implantable device comprises: a balloon implant having a neck portion and configured to receive bone cement, wherein the balloon implant has a larger size than the cavity; a shaft rigidly attached to the neck portion at a distal end; a hub attached to shaft at a proximal end; and a filler needle disposed inside the shaft and configured to provide bone cement to the balloon implant; delivering bone cement to the balloon implant; detaching the balloon implant from the shaft via a detachment device, wherein the detachment device has two or more jaws configured to radially cut the balloon implant when rotated within the shaft.
2. The method of example 1, wherein the balloon implant and the shaft are bonded together.
3. The method of example 1, wherein the hub is configured to attach to a handle of the cannula.
4. The method of example 3, wherein the hub is configured to prevent at least one of rotation and axial movement of the implantable device.
5. The method of example 4, wherein the hub contains one or more wings to overlap the handle.
6. The method of example 1, wherein the jaws of the detachment device are driven open by a mandrel inserted in a cannulated portion of the detachment device.
7. The method of example 1, wherein the filler needle includes markings indicating a depth of the needle into the balloon implant.
8. The method of example 1, wherein the filler needle is configured to provide a clearance between the filler needle and the shaft to provide venting for air while maintaining liquid within the balloon implant.
9. An implantable device for repairing a vertebral body, said implantable device comprising: a balloon implant having a neck portion and configured to receive bone cement, wherein the balloon implant has a larger size than the cavity; a shaft rigidly attached to the neck portion at a distal end; a hub attached to shaft at a proximal end; and a filler needle disposed inside the shaft and configured to provide bone cement to the balloon implant; a detachment device having two or more jaws configured to radially cut the balloon implant when rotated within the shaft.
10. The device of example 9, wherein the balloon implant and the shaft are bonded together.
11. The device of example 9, wherein the hub is configured to attach to a handle of the cannula.
12. The device of example 11, wherein the hub is configured to prevent at least one of rotation and axial movement of the implantable device.
13. The device of example 12, wherein the hub contains one or more wings to overlap the handle.
14. The device of example 9, wherein the jaws of the detachment device are driven open by a mandrel inserted in a cannulated portion of the detachment device.
15. The device of example 9, wherein the filler needle includes marking indicating a depth of the needle into the balloon implant.
16. The device of example 9, wherein the filler needle is configured to provide a clearance between the filler needle and the shaft to provide venting for air while maintaining liquid within the balloon implant.
17. An implantable device for repairing a vertebral body, said implantable device comprising: a balloon implant having a neck portion and configured to receive bone cement, wherein the balloon implant has a larger size than the cavity; a shaft attached to the neck portion at a distal end; a hub attached to shaft at a proximal end; and a filler needle disposed inside the shaft and configured to provide bone cement to the balloon implant.
18. The device of example 17, wherein the shaft further comprises a pair of steel tubes and the neck portion is disposed between the steel tubes and wherein the balloon implant is released from the shaft by retracting one of the pair of steel tubes.
19. The device of example 17, wherein the shaft is attached to the neck portion via threaded connection.
20. The device of example 19, wherein the shaft is configured to rotate to release the balloon implant from the threaded connection.

## Claims

1. An implantable device for repairing a vertebral body, said implantable device comprising:
- a balloon implant having a neck portion and configured to receive bone cement, wherein the balloon implant has a larger size than the cavity;
- a shaft rigidly attached to the neck portion at a distal end;
- a hub attached to shaft at a proximal end; and
- a filler needle disposed inside the shaft and configured to provide bone cement to the balloon implant;
- a detachment device having two or more jaws configured to radially cut the balloon implant when rotated within the shaft.

2. The device of claim 1, wherein the balloon implant and the shaft are bonded together.

3. The device of claim 1, wherein the hub is configured to attach to a handle of the cannula.

4. The device of claim 3, wherein the hub is configured to prevent at least one of rotation and axial movement of the implantable device.

5. The device of claim 4, wherein the hub contains one or more wings to overlap the handle.

6. The device of claim 1, wherein the jaws of the detachment device are driven open by a mandrel inserted in a cannulated portion of the detachment device.

7. The device of claim 1, wherein the filler needle includes marking indicating a depth of the needle into the balloon implant.

8. The device of claim 1, wherein the filler needle is configured to provide a clearance between the filler needle and the shaft to provide venting for air while maintaining liquid within the balloon implant.
